# EUROPÄISCHE PATENTSCHRIFT

(11) **EP 3 600 192 B1**
(45) Veröffentlichungstag und Bekanntmachung des Hinweises auf die Patenterteilung: **28.05.2025**
(21) Anmeldenummer: 18714231.0
(22) Anmeldetag: 27.03.2018
(51) Int. Cl.: A61F 13/15

(54) **FERTIGUNGSANLAGE FÜR HYGIENEPRODUKTE, INSBESONDERE ERWACHSENEN- ODER BABYWINDELN**
PRODUCTION SYSTEM FOR HYGIENE PRODUCTS, IN PARTICULAR ADULT OR BABY DIAPERS
INSTALLATION DE PRODUCTION DE PRODUITS D'HYGIÈNE, EN PARTICULIER DE CHANGES POUR ADULTES OU POUR BÉBÉS

(30) Priorität: 29.03.2017 DE 102017106728
(43) Veröffentlichungstag der Anmeldung: 05.02.2020
(73) Patentinhaber: Winkler + Dünnebier GmbH, 56564 Neuwied (DE)
(72) Erfinder: GEIGER, Lothar, 56626 Andernach (DE); SPURZEM, Thomas, 56727 Mayen (DE)
(74) Vertreter: Gottschald, Jan
(86) Internationale Anmeldenummer: PCT/EP2018/057790
(87) Internationale Veröffentlichungsnummer: WO 2018/178084

(56) Entgegenhaltungen:
- EP-A1- 1 287 798
- WO-A1-01/56524
- US-A1- 2011 046 772
- US-A1- 2015 176 750

## Beschreibung

Die vorliegende Erfindung betrifft eine Fertigungsanlage für Hygieneprodukte, insbesondere Erwachsenen- oder Babywindeln, mit mehreren Bearbeitungsstationen, die konfiguriert sind zur Herstellung eines ersten Produktformats.

Aus dem Stand der Technik ist eine Vielzahl von Fertigungsanlagen für Hygieneprodukte bekannt. Unter Hygieneprodukten werden körpernah eingesetzte Saugkörper verstanden, insbesondere Erwachsenen- oder Babywindeln, Inkontinenz-Vorlagen und -Pants, Damenbinden, Slipeinlagen o.dgl. Hygieneprodukte haben gemein, dass sie einen flüssigkeitsabsorbierenden Kern aufweisen, der von einer körperseitigen Materiallage (Topsheet) und einer körperfernen Materiallage (Backsheet) eingefasst ist. An diesen Mittelteil sind seitliche Befestigungselemente (Ohren bzw. Flügel) angesetzt, die zum Befestigen des Hygieneprodukts am Körper dienen.

Zur Herstellung von Hygieneprodukten, insbesondere Erwachsenen- oder Babywindeln, dienen Fertigungsanlagen mit mehreren Bearbeitungsstationen, darunter insbesondere eine Zuführvorrichtung für Bandmaterial für das Backsheet, eine Zuführvorrichtung für Bandmaterial für das Topsheet, eine Zuführvorrichtung für Bandmaterial für die seitlichen Befestigungselemente, eine Vorrichtung zum Formen des flüssigkeitsabsorbierenden Kerns und Aufsetzen desselben auf das Bandmaterial für das Backsheet etc. Die einzelnen Lagen werden bei den bekannten Fertigungsanlagen nacheinander zusammengeführt und in der Regel stoffschlüssig (durch Kleben, Ultraschallsiegeln etc.) miteinander verbunden, wodurch ein Produktstrang, im Weiteren auch fixierter Schichtaufbau genannt, entsteht, der zum Erzeugen von Einzelprodukten in vorgegebenen Abständen abgelängt und gefaltet wird. Die einzelnen Bearbeitungsstationen sind in Produktionsrichtung nacheinander in einer Fertigungsstraße angeordnet, die auf Grund der Vielzahl von Bearbeitungsstationen entweder eine sehr große Länge hat oder zweistöckig aufgebaut ist.

Entsprechende Fertigungsanlagen sind grundsätzlich dazu geeignet, Hygieneprodukte eines bestimmten Typs, beispielsweise Erwachsenenwindeln, in verschiedenen Größen herzustellen. Um von einer Windelgröße auf eine andere Windelgröße desselben Typs zu wechseln (sog. Formatwechsel), ist es allerdings notwendig, die Fertigungsstraße außer Betrieb zu nehmen und mehrere der Bearbeitungsstationen umzubauen. So unterscheidet sich beispielsweise die Größe der seitlichen Befestigungselemente bei den unterschiedlichen Größen von Erwachsenenwindeln. Auch der Abstand zwischen den vorderen Befestigungselementen (Frontears) und den hinteren Befestigungselementen (Backears) unterscheidet sich bei den unterschiedlichen Windelgrößen. Auch können sich die Größen von Backsheet, Topsheet und/oder flüssigkeitsabsorbierendem Kern bei unterschiedlichen Windelgrößen unterscheiden. Die Folge ist, dass bei einem Formatwechsel beispielsweise die Messerwelle zum Zuschneiden der seitlichen Befestigungselemente gegen eine solche einer anderen Größe ausgetauscht werden muss (der Messerwellendurchmesser für die Größen S und M beträgt beispielsweise 150 mm, der Messerwellendurchmesser für die Größen L und XL beträgt beispielsweise 210 mm). Im Zuge des Austausches muss, um einen Funktionsnachteil zu vermeiden, auch der Spalt zwischen der Messerwelle und einer gegebenenfalls vorhandenen, darunterliegenden Transferrolle des Beschleunigungssystems, durch den das jeweilige Bandmaterial in Produktionsrichtung läuft, neu eingestellt werden. Auch ist es notwendig, das System zum Beschleunigen und dadurch Beabstanden der einzelnen seitlichen Befestigungselemente auszutauschen und neu einzustellen. Entsprechendes gilt auch für eine Sammeltrommel, auf der das mit den seitlichen Befestigungselementen versehene Bandmaterial für das Topsheet mit dem mit den Kernen versehenen Bandmaterial für das Backsheet zusammengeführt wird. Auch der Spalt zwischen einer mit der Sammeltrommel zusammenwirkenden Anpressrolle zum Zusammenpressen des Schichtaufbaus muss neu eingestellt werden.

Ein Formatwechsel führt also zu relativ langen Stillstandzeiten der Fertigungsanlage und erfordert außerdem eine Vielzahl von Einstellarbeiten, die jeweils fehleranfällig sind und beim Wiederanlaufen der Fertigungsanlage nach einem Formatwechsel zunächst zu einer relativ großen Menge an Ausschuss führen können.

Unabhängig vom Vorangehenden ist es aus dem Stand der Technik, beispielsweise der EP 1 251 813 B1, bekannt, Fertigungsanlagen für Hygieneprodukte modular aufzubauen, um einige Bearbeitungsstationen als Ganzes zu Wartungs- oder Reparaturzwecken aus der Fertigungsstraße entnehmen zu können. Ein weitere System ist aus WO01/56524 bekannt.

Es ist eine Aufgabe der vorliegenden Erfindung, einen Formatwechsel bei einer Fertigungsanlage für Hygieneprodukte der eingangs genannten Art zu vereinfachen.

Die zuvor hergeleitete und aufgezeigte Aufgabe wird gelöst durch eine Fertigungsanlage mit den Merkmalen von Anspruch 1.

Die vorschlagsgemäße Fertigungsanlage für Hygieneprodukte weist mehrere Bearbeitungsstationen zur Herstellung eines ersten Produktformats und mehrere korrespondierende Bearbeitungsstationen zur Herstellung eines zweiten Produktformats auf, wobei die Bearbeitungsstationen für das erste Produktformat in einem ersten Formatwechselmodul und die Bearbeitungsstationen für das zweite Produktformat in einem separaten zweiten Formatwechselmodul zusammengefasst sind. Mit den Bearbeitungsstationen für das erste Produktformat lassen sich beispielsweise Erwachsenenwindeln der Größen S und M und mit den Bearbeitungsstationen für das zweite Produktformat Erwachsenenwindeln der Größen L und XL fertigen. Die Formatwechselmodule bilden jeweils eine funktionsfähige Einheit, die in eine Fertigungsstraße der Fertigungsanlage integriert werden kann, um das jeweilige Produktformat herzustellen. Mit anderen Worten ist zur Herstellung des ersten Produktformats das erste Formatwechselmodul in die Fertigungsstraße integriert, wohingegen zur Herstellung des zweiten Produktformats anstelle des ersten Formatwechselmoduls das zweite Formatwechselmodul in die Fertigungsstraße integriert ist. Die Fertigungsstraße und die Formatwechselmodule bilden zusammen die Fertigungsanlage.

Wesentlich ist dabei die Erkenntnis, dass sich durch Vorsehen mehrerer Formatwechselmodule, die jeweils mehrere formatabhängige Bearbeitungsstationen zusammenfassen und als Ganzes austauschbar sind, Stillstandzeiten bei einem Formatwechsel erheblich reduziert werden können. So ist es bei der vorschlagsgemäßen Fertigungsanlage nicht mehr notwendig, einzelne Bearbeitungsstationen umzubauen, beispielsweise eine andere Messerwelle vorzusehen, und Einstellungen vorzunehmen, beispielsweise Einstellungen von Spalten innerhalb einzelner Bearbeitungsstationen oder Abstände zwischen Bearbeitungsstationen, da zum Formatwechsel bereits ein entsprechendes weiteres, vollständig eingestelltes Formatwechselmodul bereitsteht. Dabei kann auch, während das erste Formatwechselmodul noch in die Fertigungsstraße integriert ist und entsprechende Hygieneprodukte des ersten Produktformats produziert werden, das zweite Formatwechselmodul, das sich außerhalb der Fertigungsstraße befindet, optimal gewartet, repariert, eingestellt und/oder getestet werden, so dass die Fertigungsstraße nur für die Zeit des Austausches des ersten Formatwechselmoduls gegen das zweite Formatwechselmodul stillstehen muss. Die Fertigungsstraße kann unmittelbar nach dem Austausch wieder angefahren werden und kann sofort Hygieneprodukte des zweiten Produktformats herstellen. Da das zweite Formatwechselmodul bereits vor seiner Integrierung in die Fertigungsstraße optimal eingestellt werden konnte, entfallen auch nachträgliche Einstellarbeiten zur Nachjustierung während des Anlaufens der Fertigungsstraße, was auch das Risiko von entsprechendem Materialausschuss erheblich reduziert.

Es sei darauf hingewiesen, dass die vorschlagsgemäß in die Fertigungsstraße integrierbaren Formatwechselmodule, von denen grundsätzlich auch mehr als zwei vorgesehen sein können, nicht zwingend alle formatabhängigen Bearbeitungsstationen der Fertigungsstraße aufweisen müssen. So kann beispielsweise eine Kernformvorrichtung zum Herstellen und Formen der flüssigkeitsabsorbierenden Kerne und Auflegen derselben auf das Bandmaterial der körperfernen Lage (Backsheet) vom jeweiligen Formatwechselmodul unabhängig angeordnet sein, wobei vorzugsweise alle übrigen formatabhängigen Bearbeitungsstationen, die gemäß Stand der Technik einen Umbau erfordert hätten, in einem Formatwechselmodul zusammengefasst sind. Entsprechend muss zum Zwecke des Formatwechsels die Kernformvorrichtung separat umgebaut werden. Es hat sich aber gezeigt, dass sich gerade die Kernformvorrichtung relativ einfach austauschen und justieren lässt. Der Verzicht auf das Integrieren der Kernformvorrichtung in ein jeweiliges Formatwechselmodul hat den Vorteil, dass die einzelnen Formatwechselmodule geringere Abmessungen und ein geringeres Gewicht haben, wodurch diese leichter handhabbar sind.

Es hat sich herausgestellt, dass die meisten und zeitaufwendigsten Arbeiten bei einem Formatwechsel bei der Schneidevorrichtung für die seitlichen Befestigungselemente oder Zwischenprodukte und bei der Lagenverbindevorrichtung, mit der die körperseitige Lage (Topsheet) und die körperferne Lage (Backsheet) und eventuell damit verbundene weitere Lagen zusammengeführt werden, erforderlich sind. Entsprechend ist bei der Ausgestaltung der Fertigungsanlage gemäß Anspruch 1 vorgesehen, dass die Formatwechselmodule jeweils sowohl die Schneidevorrichtung für die seitlichen Befestigungselemente oder Zwischenprodukte als auch die Lagenverbindevorrichtung aufweisen. Die Lagenverbindevorrichtung weist insbesondere eine Sammeltrommel (Anspruch 2 und eine Anpressrolle (Anspruch 3) auf.

Zusätzlich kann bei der Ausgestaltung gemäß Anspruch 4 das erste und zweite Formatwechselmodul im Abschnitt zwischen der Schneidevorrichtung und der Lagenverbindevorrichtung noch eine Handhabungsvorrichtung aufweisen, die jeweils dazu dient, die seitlichen Befestigungselemente oder Zwischenprodukte in Produktionsrichtung zu beschleunigen und/oder einzelne Befestigungselemente oder Zwischenprodukte zu drehen, um ihre Ausrichtung für das nachfolgende Verbinden mit dem Bandmaterial der körperseitigen Lage anzupassen.

Das jeweilige Formatwechselmodul kann außerdem als Bearbeitungsstation für das jeweilige Produktformat noch eine erste Zuführvorrichtung für Bandmaterial für die seitlichen Befestigungselemente und/oder eine zweite Zuführvorrichtung für Bandmaterial für die körperseitige Lage und/oder eine dritte Zuführvorrichtung für Bandmaterial für die körperferne Lage und/oder eine vierte Zuführvorrichtung für Querbänder sowie eine entsprechende Schneidevorrichtung für die Querbänder und/oder eine Übergabevorrichtung für den Schichtaufbau an nachfolgende Bearbeitungsstationen aufweisen (Anspruch 5).

Weitere Bearbeitungsstationen der Fertigungsanlage, die insbesondere nicht Bestandteil des jeweiligen Formatwechselmoduls sind, sind jeweils in den Ansprüchen 6 bis 10 definiert.

Bei der Ausgestaltung der vorschlagsgemäßen Fertigungsanlage gemäß Anspruch 11 weist das jeweilige Formatwechselmodul einen Modulrahmen auf, in den die entsprechenden formatabhängigen Bearbeitungsstationen jeweils eingebaut sind. Die Formatwechselmodule der vorschlagsgemäßen Fertigungsanlage können auch, insbesondere am Modulrahmen, Bewegungseinheiten aufweisen, mit denen sich die Formatwechselmodule relativ zur übrigen Fertigungsstraße als Ganzes bewegen lassen, sowie zusätzlich oder alternativ eine Kupplungseinheit zum lösbaren Verbinden (Zusammenkuppeln) mit der übrigen Fertigungsstraße (Anspruch 12).

Es gibt nun eine Vielzahl von Möglichkeiten, die vorschlagsgemäße Fertigungsanlage auszugestalten und weiterzubilden. Diesbezüglich sei einerseits verwiesen auf die dem Anspruch 1 nachgeordneten Ansprüche, andererseits auf die Beschreibung von Ausführungsbeispielen in Verbindung mit der Zeichnung. In der Zeichnung zeigt:
- Fig. 1: ein Ausführungsbeispiel eines Hygieneprodukts, das mit der vorschlagsgemäßen Fertigungsanlage herstellbar ist,
- Fig. 2: eine schematische Darstellung eines Herstellungsprozesses für ein Hygieneprodukt gemäß Fig. 1,
- Fig. 3: eine schematische Darstellung eines alternativen Herstellungsprozesses für ein Hygieneprodukt gemäß Fig. 1,
- Fig. 4a): eine schematische Seitenansicht einer Fertigungsanlage zur Herstellung eines Hygieneprodukts gemäß Fig. 1,
- Fig. 4b): eine schematische Draufsicht auf die Fertigungsanlage gemäß Fig. 4a) und
- Fig. 5: eine schematische Darstellung des Aufbaus von Formatwechselmodulen für die Fertigungsanlage gemäß Fig. 4a) und b).

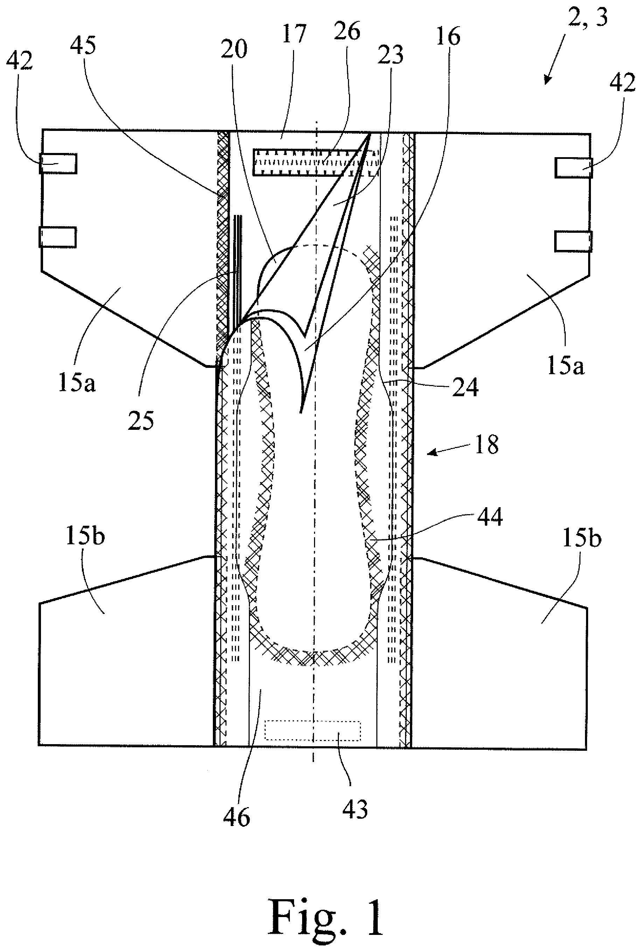

Fig. 1 zeigt schematisch den Aufbau eines Hygieneprodukts 2, hier beispielhaft in Form einer Erwachsenenwindel 3, welches mit einer vorschlagsgemäßen Fertigungsanlage 1 hergestellt werden kann.

Das Hygieneprodukt 2 besteht im Wesentlichen aus einem mehrlagigen Mittelteil 46 (Chassis) und in einem hinteren Abschnitt angesetzten seitlichen Befestigungselementen 15a (Backears) und in einem vorderen Abschnitt angesetzten seitlichen Befestigungselementen 15b (Frontears).

Der Mittelteil 46 weist eine obere und im bestimmungsgemäßen Gebrauch körperseitige Lage 16 (Topsheet) und eine untere und im bestimmungsgemäßen Gebrauch körperferne Lage 17 (Backsheet) auf. Zwischen dem das Topsheet bildenden Bandmaterial 16 und dem das Backsheet bildenden Bandmaterial 17 weist das Hygieneprodukt 2 einen flüssigkeitsabsorbierenden Kern 20 und vorzugsweise eine Flüssigkeitsaufnahme- und/oder -verteilungslage 23 (Acquisition Layer) auf, wobei rund um den Kern 20 ein Andrückbereich 44 vorgesehen ist, in welchem die Lagen des Mittelteils 46 zusammengepresst sind.

An den zu den Befestigungselementen 15a und 15b weisenden Rändern des Mittelteils 46 ist das Topsheet 16 an beiden Längsseiten noch mit einem klappenförmigen Auslaufschutz 24 (Upstanding Cuffs) versehen, der dazu dient, im bestimmungsgemäß angelegten Zustand Flüssigkeit zurückzuhalten. Außerdem sind im Mittelteil 46 elastische Längsbänder 25 und elastische Querbänder 26 vorgesehen, um einen optimalen Sitz des Hygieneprodukts 2 am Körper des Benutzers zu gewährleisten.

Die seitlichen Befestigungselemente 15a und 15b sind hier und vorzugsweise zwischen dem Bandmaterial 16 des Topsheets und dem Bandmaterial 17 des Backsheets in einem Verbindungsbereich 45 fixiert, was mittels Heißkleben, Ultraschallsiegeln und/oder Heißprägen erfolgen kann. Die hinteren seitlichen Befestigungselemente 15a weisen außerdem noch Befestigungsstreifen 42 auf, insbesondere in Form von Klebe- und/oder Klettstreifen. Bei bestimmten Produktgrößen kann zu Befestigungszwecken, insbesondere zum Zusammenwirken mit einem der Befestigungsstreifen 42, am Mittelteil 46 noch eine Frontalbefestigungsfläche 43 (Frontal Tape) vorgesehen sein, die als Klebe- und/oder Klettstreifen ausgebildet sein kann.

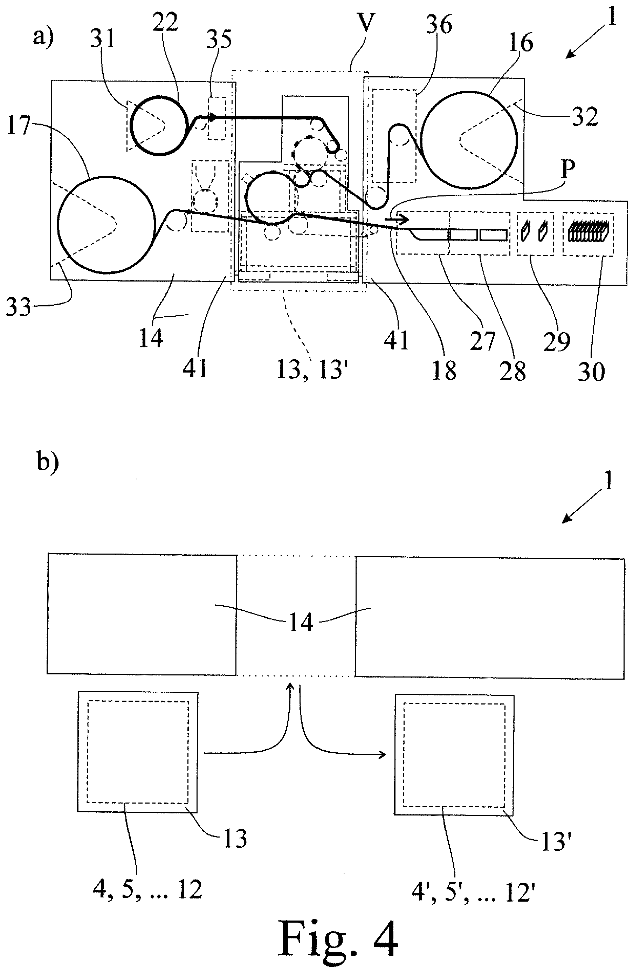

Das zuvor anhand von Fig. 1 beispielhaft beschriebene Hygieneprodukt 2 bzw. der entsprechende Schichtaufbau 18 kann in einer Fertigungsanlage 1 hergestellt werden, wie sie beispielhaft in den Figuren 4a), 4b) und 5 dargestellt ist.

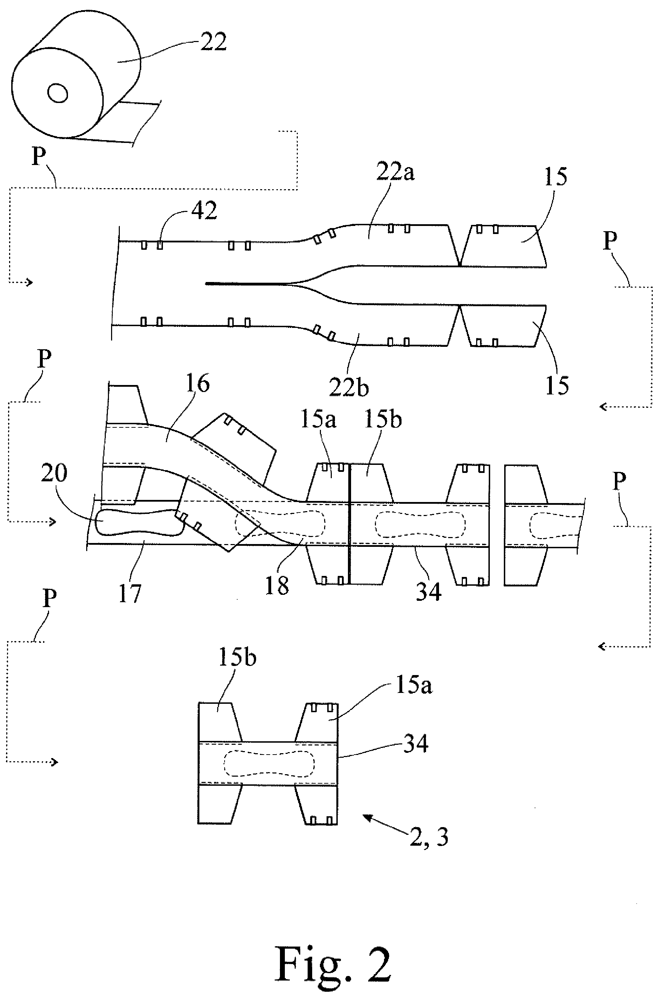

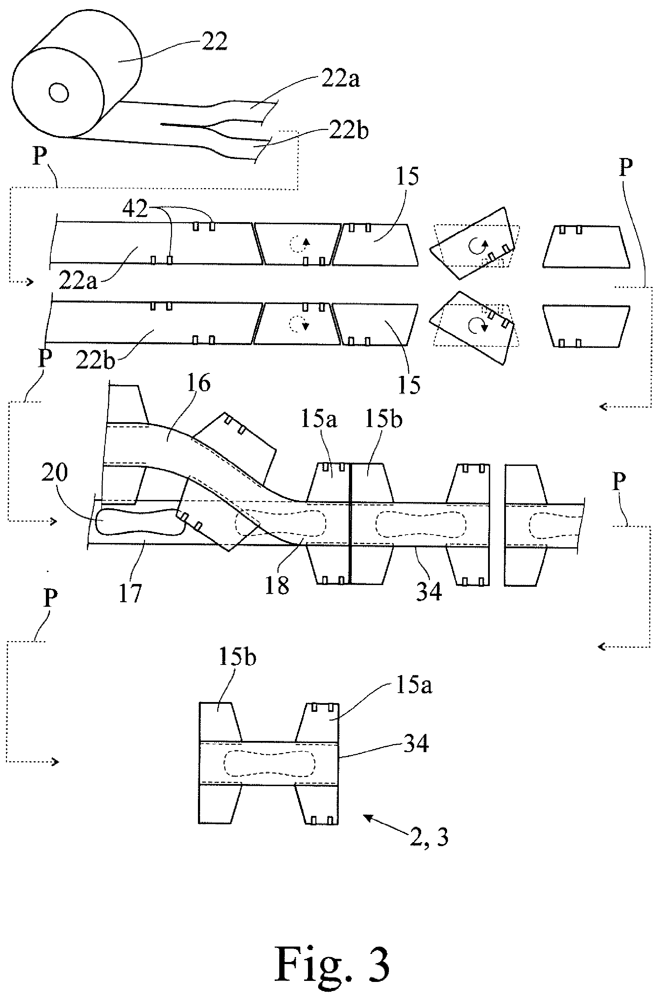

Ein beispielhafter Fertigungsprozess ist in zwei alternativen Ausführungsbeispielen in den Fig. 2 und 3 dargestellt.

Die vorschlagsgemäße Fertigungsanlage 1 weist eine Vielzahl von Bearbeitungsstationen 4 bis 12, 4' bis 12' und 27 bis 30 auf, von denen die Bearbeitungsstationen 4 bis 12 und 4' bis 12' jeweils formatabhängige Bearbeitungsstationen sind, d. h. Bearbeitungsstationen, die zur Herstellung eines bestimmten Produktformats (einer bestimmten Produktgröße) ausgelegt sind und bei einem Formatwechsel ausgetauscht werden. Die Bearbeitungsstationen 27 bis 30 sind dagegen formatunabhängig und müssen daher bei einem Formatwechsel nicht ausgetauscht werden.

Die Bearbeitungsstationen 4 bis 12, die zur Herstellung eines ersten Produktformats konfiguriert sind, sind in einem ersten Formatwechselmodul 13 zusammengefasst und die Bearbeitungsstationen 4' bis 12' sind in einem zweiten, separaten Formatwechselmodul 13' zusammengefasst. Das erste Formatwechselmodul 13 und das zweite Formatwechselmodul 13' können je nach zu produzierendem Produktformat abwechselnd in eine Fertigungsstraße 14 der Fertigungsanlage 1 integriert werden. Sollen Hygieneprodukte 2 des ersten Produktformats hergestellt werden, ist das erste Formatwechselmodul 13 in die Fertigungsstraße 14 integriert und das zweite Formatwechselmodul 13' ist außerhalb der Fertigungsstraße 14 angeordnet. Im umgekehrten Fall, wenn das zweite Produktformat hergestellt werden soll, wird das erste Formatwechselmodul 13 gegen das zweite Formatwechselmodul 13' ausgetauscht.

Die beiden Formatwechselmodule 13 und 13' haben insbesondere denselben Aufbau, weisen also korrespondierende Bearbeitungsstationen bzw. dieselben Arten von Bearbeitungsstationen auf, wobei sich aber insbesondere die Größe der korrespondierenden Bearbeitungsstationen, die Abstände zwischen benachbarten korrespondierenden Bearbeitungsstationen und/oder die Bauteilabstände innerhalb der korrespondierenden Bearbeitungsstationen bei den beiden Formatwechselmodulen 13 und 13' unterscheiden, um die gewünschten unterschiedlichen Produktformate herstellen zu können. Bei dem ersten Produktformat kann es sich beispielsweise um Erwachsenenwindeln 3 in den Größen S und M und bei dem zweiten Produktformat um Erwachsenenwindeln 3 in den Größen L und XL handeln. Die Abmessungen eines Hygieneprodukts 2 in der Größe S und eines Hygieneprodukts 2 in der Größe M können sich zwar etwas unterscheiden, wobei die Unterschiede aber so gering sind, dass keine oder zumindest keine nennenswerten Änderungen innerhalb des Formatwechselmoduls 13 erforderlich sind. Entsprechendes gilt auch für die Unterschiede in den Abmessungen der Größen L und XL, die ebenfalls keine oder zumindest keine nennenswerten Änderungen in dem Formatwechselmodul 13' erfordern. Bei den seitlichen Befestigungselementen 15a bzw. 15b unterscheiden sich bei den Größen S und M die Gesamtlängen um beispielsweise 40 mm, bei den Größen L und XL sogar nur um beispielsweise 15 mm, was keinen Austausch des Formatwechselmoduls 13 bzw. 13' erfordert, wohingegen sich bei den Größen M und L die Gesamtlängen um beispielsweise 90 mm unterscheiden können, was dann einen Austausch des Formatwechselmoduls 13 bzw. 13' erfordert.

Die Formatwechselmodule 13 und 13' sowie ggf. weitere hier nicht dargestellte korrespondierende Formatwechselmodule sind insbesondere voll funktionsfähig, wenn sie in die Fertigungsstraße 14 integriert werden. Es ist insbesondere lediglich notwendig, das jeweils integrierte Formatwechselmodul 13 bzw. 13' elektrisch, pneumatisch und/oder hydraulisch anzuschließen. Das jeweilige Formatwechselmodul 13 bzw. 13' ist insbesondere auch im nicht in die Fertigungsstraße 14 integrierten Zustand voll funktionsfähig, so dass hier jederzeit auch außerhalb der Fertigungsstraße ein Funktionstest durchgeführt werden kann.

Bei dem in den Figuren 4a), 4b) und 5 dargestellten Ausführungsbeispiel weisen alle Formatwechselmodule 13 und 13' jeweils folgende Bearbeitungsstationen bzw. Arten von Bearbeitungsstationen auf:
- eine Schneidevorrichtung 4, 4', insbesondere mit mindestens einer Messerwelle und jeweils einer korrespondierenden Gegenwelle bzw. einem korrespondierenden Gegenmesser, konfiguriert zur Formgebung seitlicher Befestigungselemente 15a, 15b des Hygieneprodukts 2 oder von Zwischenprodukten 15, aus denen die seitlichen Befestigungselemente 15a, 15b erzeugt werden,
- eine Lagenverbindevorrichtung 5, 5' konfiguriert zum Zusammenführung zumindest von einem eine körperseitige Lage bildenden Bandmaterial 16, einem die körperferne Lage bildenden Bandmaterial 17 sowie der seitlichen Befestigungselemente 15a, 15b oder Zwischenprodukte 15 und Verbinden zu einem fixierten Schichtaufbau 18, wobei die seitlichen Befestigungselemente 15a, 15b oder Zwischenprodukte 15 mit dem die körperseitige Lage bildenden Bandmaterial 16 und/oder dem die körperferne Lage bildenden Bandmaterial 17 insbesondere stoffschlüssig, vorzugsweise mittels Applizieren von Heißleim, Ultraschallsiegeln und/oder Heißprägen, kraftschlüssig und/oder formschlüssig verbindbar sind, sowie vorzugsweise
- eine Handhabungsvorrichtung 6, 6', die konfiguriert ist, Zwischenprodukte 15, aus denen seitliche Befestigungselemente 15a und 15b entstehen, in Produktionsrichtung P, beispielsweise unter Verwendung einer Transferrolle, von der die jeweiligen Produkte/Elemente 15, 15a, 15b abgegriffen werden, zu beschleunigen und insbesondere in Produktionsrichtung P voneinander zu beabstanden und/oder jedes zweite der Zwischenprodukte 15 um insbesondere 180° zu drehen, und/oder
- eine erste Zuführvorrichtung 7, 7' konfiguriert zum Zuführen von Bandmaterial 22 für die seitlichen Befestigungselemente 15a, 15b oder Zwischenprodukte 15, insbesondere in Form von zwei Bandmaterialsträngen 22a, 22b, zur Schneidevorrichtung 4, 4' und/oder
- eine zweite Zuführvorrichtung 8, 8' konfiguriert zum Zuführen des die körperseitige Lage bildenden Bandmaterials 16 zur Lagenverbindevorrichtung 5, 5', wobei das die körperseitige Lage bildende Bandmaterial 16, insbesondere zumindest mit einer Flüssigkeitsaufnahme- und -verteilungslage 23 und/oder mit Einem Auslaufschutz 24 und/oder mit einer Zwischenlage versehen ist und/oder
- eine dritte Zuführvorrichtung 9, 9' konfiguriert zum Zuführen des die körperferne Lage bildenden Bandmaterials 17 zur Lagenverbindevorrichtung 5, 5', wobei das die körperferne Lage bildende Bandmaterial 17 insbesondere mit flüssigkeitsabsorbierenden Kernen 20 und/oder mit elastischen Längsbändern 25 versehen ist, und/oder
- eine vierte Zuführvorrichtung 10, 10' konfiguriert zum Zuführen von elastischen Querbändern 26 sowie insbesondere eine Schneidevorrichtung 11, 11', konfiguriert zum Zuschneiden der elastischen Querbänder 26 und/oder
- eine Übergabevorrichtung 12, 12' konfiguriert zum Übergeben des fixierten Schichtaufbaus 18 an eine weitere Bearbeitungsstation 27, 28, ...33, die insbesondere nicht Bestandteil des jeweiligen Formatwechselmoduls 13, 13' ist.

Neben den vorgenannten formatabhängigen Bearbeitungsstationen 4 bis 12 bzw. 4' bis 12' sind ferner als Bestandteil der übrigen Fertigungsstraße 14 noch eine oder mehrere der folgenden weiteren Bearbeitungsstationen vorgesehen:
- eine erste Faltvorrichtung 27, die zum Längsfalten des fixierten Schichtabbaus konfiguriert ist, und/oder
- eine Ablängvorrichtung 28, die zum Ablängen des fixierten Schichtaufbaus 18 konfiguriert ist, und/oder
- eine zweite Faltvorrichtung 29, die zum Querfalten von aus dem fixierten Schichtaufbau 18 erzeugten Einzelprodukten 34 konfiguriert ist, und/oder
- eine Stapelvorrichtung 30, die zum Stapeln und insbesondere Verpacken von aus dem fixierten Schichtaufbau 18 erzeugten Einzelprodukten 34 konfiguriert ist.

Die zuvor genannten weiteren Bearbeitungsstationen 27 bis 30 sind dabei in Produktionsrichtung P hinter dem Aufnahmeabschnitt für die Formatwechselmodule 13 und 13' angeordnet. Dagegen sind eine oder mehrere der folgenden weiteren Bearbeitungsstationen bezogen auf die Produktionsrichtung P vor dem Aufnahmeabschnitt für die Formatwechselmodule 13 und 13' angeordnet:
- eine erste Bereitstellungsvorrichtung 31, die konfiguriert ist zum Zuführen von Bandmaterial 22 für die Zwischenprodukte 15, aus denen die Befestigungselemente 15a und15b entstehen, zum jeweiligen Formatwechselmodul 13 bzw. 13' und insbesondere zur ersten Zuführvorrichtung 7 bzw. 7' des jeweiligen Formatwechselmoduls 13 bzw. 13', wobei die erste Breitstellungsvorrichtung 31 insbesondere eine Längszerteilungsvorrichtung 35 zum Zerteilen des Bandmaterials 22 in zwei separate Bandmaterialstränge 22a und 22b umfasst, und/oder
- eine zweite Bereitstellungsvorrichtung 32, die konfiguriert ist zum Zuführen von die körperseitige Lage bildendem Material 16 zum jeweiligen Formatwechselmodul 13 bzw. 13' und insbesondere zur zweiten Zuführvorrichtung 8 bzw. 8' des jeweiligen Formatwechselmoduls 13 bzw. 13', wobei die zweite Bereitstellungsvorrichtung 32 insbesondere eine Schichtungsvorrichtung 36 zum Zusammenführen des die körperseitige Lage bildenden Bandmaterials 16 und einer Flüssigkeitsaufnahme- und -verteilungslage 23 (Acquisition Layer) und/oder einem Auslaufschutz 24 (Upstanding Cuffs) und/oder einer Zwischenlage umfasst, und/oder
- eine dritte Bereitstellungsvorrichtung 33, die konfiguriert ist zum Zuführen von die körperferne Lage bildendem Bandmaterial 17 zum jeweiligen Formatwechselmodul 13 bzw. 13' und insbesondere zur dritten Zuführvorrichtung 9, 9' des jeweiligen Formatwechselmoduls 13 bzw. 13', wobei die dritte Bereitstellungsvorrichtung 33 insbesondere eine Kernformvorrichtung 37 umfasst, die konfiguriert ist zum Herstellen und Formen flüssigkeitsabsorbierender Kerne 20 und Auflegen der Kerne 20 auf das die körperferne Lage bildende Bandmaterial 17.

Die Lagenverbindevorrichtung 5 bzw. 5' des jeweiligen Formatwechselmoduls 13 bzw. 13' weist eine Sammeltrommel 19 auf, die konfiguriert ist, das die die körperseitige Lage bildende Bandmaterial 16, das insbesondere zumindest mit den Zwischenprodukten 15 für die seitlichen Befestigungselemente 15a und 15b versehen ist, mit dem die körperferne Lage bildenden Bandmaterial 17, das insbesondere zumindest mit den flüssigkeitsabsorbierenden Kernen 20 versehen ist, zusammenzuführen. Die Sammeltrommel 19 ist dabei insbesondere konfiguriert, das das Topsheet bildende Bandmaterial 16 anzusaugen, wozu die Sammeltrommel 19 beispielsweise umfänglich angeordnete Öffnungen (nicht dargestellt) aufweist und mit einer Unterdruckerzeugungseinrichtung (nicht dargestellt) in Fluidverbindung steht, die insbesondere nicht Teil des jeweiligen Formatwechselmoduls 13 bzw. 13' ist.

Die Lagenverbindevorrichtung 5 bzw. 5' weist hier ferner eine Anpressrolle 21 auf, die mit der Sammeltrommel 19 derart zusammenwirkt, dass der Schichtaufbau 18 zumindest abschnittsweise zwischen Sammeltrommel 19 und Anpressrolle 21, insbesondere in einem Andrückbereich 44 um den Kern 20 herum, zusammengepresst wird.

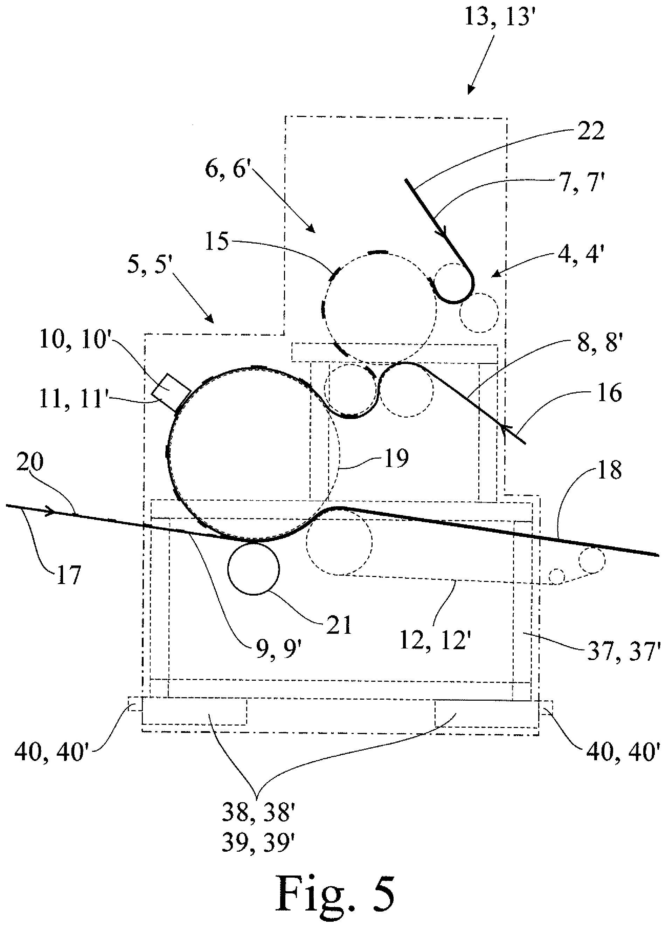

Ferner ist in Fig. 5 auch zu erkennen, dass jedes der Formatwechselmodule 13 und 13' hier und vorzugsweise jeweils einen Modulrahmen 37 bzw. 37' aufweist, in den die Bearbeitungsstationen 4 bis 12 bzw. 4' bis 12' für das jeweilige Produktformat eingebaut sind. Die Modulrahmen 37 und 37' haben dabei insbesondere dieselben Abmessungen und sind mit einer Bewegungseinheit 38 bzw. 38' in Form von Luftgleitkissen 39 bzw. 39' und einer Kupplungseinheit 40 bzw. 40' zum Zusammenkuppeln mit entsprechenden in der Fertigungsstraße 14 vorgesehenen Gegenstücken 41 versehen.

In den Fig. 2 und 3 ist der prinzipielle Ablauf der Herstellung von Hygieneprodukten 2 mit der vorschlagsgemäßen Fertigungsanlage 1 dargestellt.

Im ersten Ausführungsbeispiel (Fig. 2) wird Bandmaterial 22 für die seitlichen Befestigungselemente 15a und 15b bzw. das vorherige Zwischenprodukt 15 in Form einer Endlosrolle bereitgestellt. In der weiteren Folge wird das Bandmaterial 22 mittels einer Längszerteilungsvorrichtung 35 in zwei Stränge 22a und 22b aufgeteilt, wobei in der Schneidevorrichtung 4 bzw. 4' die Zwischenprodukte 15 für die seitlichen Befestigungselemente 15a und 15b geformt bzw. zugeschnitten werden, wobei in dieser Variante etwas Verschnitt anfällt (Low Waste-Variante), der abgesaugt werden kann. Die Zwischenprodukte 15 werden anschließend insbesondere stoffschlüssig mit dem Bandmaterial 16 der körperseitigen Lage (Topsheet) verbunden, beispielsweise mittels Leim und/oder Ultraschall und/oder Prägen, wobei dieses Bandmaterial 16 anschließend mit dem Bandmaterial 17 der körperfernen Lage (Backsheet), auf das zuvor die flüssigkeitsabsorbierenden Kerne 20 aufgelegt wurden, zusammengeführt wird. Das Zusammenführen geschieht dabei auf der Sammeltrommel 19, wobei der entstandene Schichtaufbau bzw. Produktstrang 18 im weiteren Verlauf zwischen Sammeltrommel 19 und Anpressrolle 21 um die Kerne 20 herum zusammengedrückt wird. Der Schichtaufbau 18, in welchen auch noch eine Flüssigkeitsaufnahme- und/oder -verteilungslage 23 (Acquisition Layer) sowie elastische Längsbänder 25 und elastische Querbänder 26 integriert sein können, wird anschließend in der Ablängvorrichtung 28 zerteilt, wodurch ein Einzelprodukt 34 entsteht. Die Trennung zwischen dem Einzelprodukt 34 und dem vorangehenden schichtförmigen Strang 18 verläuft dabei quer zur Produktionsrichtung P durch die Zwischenprodukte 15, wodurch die Zwischenprodukte 15 aufgeteilt werden in die seitlichen Befestigungselemente 15a und 15b, welche bereits anhand von Fig. 1 beschrieben wurden. Vor dem Ablängen kann noch eine Längsfaltung und anschließend eine Querfaltung durchgeführt werden (in Fig. 2 nicht dargestellt). Die fertigen Einzelprodukte 34 können anschließend verpackt werden.

Das zweite Ausführungsbeispiel (Fig. 3) unterscheidet sich von der Variante in Fig. 2 dadurch, dass die Zwischenprodukte 15 für die späteren seitlichen Befestigungselemente 15a und 15b verschnittfrei zugeschnitten werden (Zero Waste-Variante). Dies erfordert nach dem Zuschneiden in der Schneidevorrichtung 4 bzw. 4' ein Drehen jedes zweiten Zwischenprodukts 15 um 180° um eine zum Zwischenprodukt 15 senkrechte Achse (die Achse kann auch anders verlaufen, solange das Zwischenprodukt anschließend wie die dazu benachbarten Zwischenprodukte ausgerichtet ist). Der weitere Verlauf des Verfahrens ist mit dem anhand von Fig. 2 beschriebenen identisch.

Schließlich ist noch ein weiterer Unterschied zwischen den Ausführungsbeispielen in Fig. 2 und Fig. 3, dass gemäß Fig. 2 die Befestigungsstreifen 42 (z.B. Klebestreifen) vor dem Erzeugen der beiden Einzelstränge 22a und 22b an das Bandmaterial 22 angebracht werden, wohingegen gemäß Fig. 3 erst die Längszerteilung in die beiden Einzelstränge 22a und 22b durchgeführt wird und erst danach die Befestigungsstreifen 42 angebracht werden.

### Bezugszeichenliste

- 1: Fertigungsanlage
- 2: Hygieneprodukt
- 3: Erwachsenen- oder Babywindel
- 4, 4': Schneidevorrichtung für seitliche Befestigungselemente
- 5, 5': Lagenverbindevorrichtung
- 6, 6': Handhabungsvorrichtung für die seitlichen Befestigungselemente
- 7, 7': Erste Zuführvorrichtung für Bandmaterial für die seitlichen Befestigungselemente
- 8, 8': Zweite Zuführvorrichtung für Bandmaterial für die körperseitige Lage
- 9, 9': Dritte Zuführvorrichtung für Bandmaterial für die körperferne Lage
- 10, 10': Vierte Zuführvorrichtung für Querbänder
- 11, 11': Schneidevorrichtung für die Querbänder
- 12, 12': Übergabevorrichtung für den Schichtaufbau
- 13: Erstes Formatwechselmodul
- 13': Zweites Formatwechselmodul
- 14: Fertigungsstraße
- 15: Zwischenprodukte, aus denen seitliche Befestigungselemente erzeugt werden
- 15a, 15b: Seitliche Befestigungselemente
- 16: Bandmaterial für die körperseitige Lage bzw. Topsheet
- 17: Bandmaterial für körperferne Lage bzw. Backsheet
- 18: Schichtaufbau, bzw. Produktstrang
- 19: Sammeltrommel
- 20: Flüssigkeitsabsorbierende Kerne
- 21: Anpressrolle
- 22: Bandmaterial für die seitlichen Befestigungselemente bzw. Zwischenprodukte
- 22a, 22b: Bandmaterialstränge für die seitlichen Befestigungselemente bzw. Zwischenprodukte
- 23: Flüssigkeitsaufnahme- und/oder -verteilungslage (Acquisition/Distribution Layer, ADL)
- 24: Auslaufschutz (Upstanding Cuffs)
- 25: Längsbänder
- 26: Querbänder
- 27: Erste Faltvorrichtung zum Längsfalten
- 28: Ablängvorrichtung zum Ablängen
- 29: Zweite Faltvorrichtung zum Querfalten
- 30: Stapelvorrichtung zum Stapeln und insbesondere Verpacken
- 31: Erste Bereitstellungsvorrichtung für Bandmaterial für die seitlichen Befestigungselemente bzw. Zwischenprodukte
- 32: Zweite Bereitstellungsvorrichtung für Bandmaterial für die körperseitige Lage
- 33: Dritte Bereitstellungsvorrichtung für Bandmaterial für die körperferne Lage
- 34: Einzelprodukte
- 35: Längszerteilungsvorrichtung zum Erzeugen der Bandmaterialstränge für die seitlichen Befestigungselemente bzw. Zwischenprodukte
- 36: Schichtungsvorrichtung
- 37, 37': Modulrahmen
- 38, 38': Bewegungseinheit
- 39, 39': Luftgleitkissen
- 40, 40': Kupplungseinheit
- 41: Gegenstücke für Kupplungseinheit
- 42: Befestigungsstreifen (z.B. Klebestreifen)
- 43: Frontalbefestigungsfläche (Frontaltape)
- 44: Andrückbereich um Kern
- 45: Verbindungsbereich zwischen seitlichem Befestigungselement und körperseitiger und körperferner Lage
- 46: Mittelteil (Chassis)
- P: Produktionsrichtung

## Patentansprüche

1. Fertigungsanlage (1) für Hygieneprodukte (2), insbesondere Erwachsenen- oder Babywindeln (2),
- mit mehreren Bearbeitungsstationen (4, 5, ...12), die konfiguriert sind zur Herstellung eines ersten Produktformats, und
- mit mehreren Bearbeitungsstationen (4', 5', ...12'), die konfiguriert sind zur Herstellung eines zweiten Produktformats,
wobei die Bearbeitungsstationen (4, 5, ...12) für das erste Produktformat in einem ersten Formatwechselmodul (13) zusammengefasst sind und die Bearbeitungsstationen (4', 5',...12') für das zweite Produktformat in einem zweiten Formatwechselmodul (13') zusammengefasst sind und
wobei das erste Formatwechselmodul (13) und das zweite Formatwechselmodul (13') abwechselnd in eine Fertigungsstraße (14) der Fertigungsanlage (1) integrierbar sind,
wobei das erste Formatwechselmodul (13) als Bearbeitungsstationen (4, 5) für das erste Produktformat und das zweite Formatwechselmodul (13') als Bearbeitungsstationen (4', 5') für das zweite Produktformat jeweils aufweist:
- eine Schneidevorrichtung (4, 4') konfiguriert zur Formgebung seitlicher Befestigungselemente (15a, 15b) des Hygieneprodukts (2) oder von Zwischenprodukten (15), aus denen die seitlichen Befestigungselemente (15a, 15b) erzeugt werden, und
- eine Lagenverbindevorrichtung (5, 5') konfiguriert zum Zusammenführung zumindest von einem eine körperseitige Lage bildenden Bandmaterial (16), einem die körperferne Lage bildenden Bandmaterial (17) sowie der seitlichen Befestigungselemente (15a, 15b) oder Zwischenprodukte (15) und Verbinden zu einem fixierten Schichtaufbau (18), wobei die seitlichen Befestigungselemente (15a, 15b) oder Zwischenprodukte (15) mit dem die körperseitige Lage bildenden Bandmaterial (16) und/oder dem die körperferne Lage bildenden Bandmaterial (17) insbesondere stoffschlüssig, vorzugsweise mittels Applizieren von Heißleim, Ultraschallsiegeln und/oder Heißprägen, kraftschlüssig und/oder formschlüssig verbindbar sind,
wobei die Fertigungsanlage (1) als weitere Bearbeitungsstation (27), die nicht Bestandteil des jeweiligen Formatwechselmoduls (13, 13') ist,
- eine erste Faltvorrichtung (27) aufweist, die zum Längsfalten des fixierten Schichtaufbaus (18) konfiguriert ist, und/oder
- eine Ablängvorrichtung (28) aufweist, die zum Ablängen des fixierten Schichtaufbaus (18) konfiguriert ist, und/oder
- eine zweite Faltvorrichtung (29) aufweist, die zum Querfalten von aus dem fixierten Schichtaufbau (18) erzeugten Einzelprodukten (34) konfiguriert ist, und/oder
- eine Stapelvorrichtung (30) aufweist, die zum Stapeln und insbesondere Verpacken von aus dem fixierten Schichtaufbau (18) erzeugten Einzelprodukten (34) konfiguriert ist.

2. Fertigungsanlage (1) nach Anspruch 1, **dadurch gekennzeichnet, dass** die Lagenverbindevorrichtung (5, 5') eine Sammeltrommel (19) aufweist, die konfiguriert ist, das die körperseitige Lage bildende Bandmaterial (16), das insbesondere zumindest mit den seitlichen Befestigungselementen (15a, 15b) oder Zwischenprodukten (15) versehen ist, mit dem die körperferne Lage bildenden Bandmaterial (17), das insbesondere zumindest mit flüssigkeitsabsorbierenden Kernen (20) versehen ist, zusammenzuführen, wobei die Sammeltrommel (19) insbesondere konfiguriert ist, das die körperseitige Lage bildende Bandmaterial (16) anzusaugen.

3. Fertigungsanlage (1) nach Anspruch 2, **dadurch gekennzeichnet, dass** die Lagenverbindevorrichtung (5, 5') eine Anpressrolle (21) aufweist, die mit der Sammeltrommel (19) derart zusammenwirkt, dass der Schichtaufbau (18) zumindest abschnittsweise zwischen Sammeltrommel (19) und Anpressrolle (21), insbesondere zumindest um die Kerne (20) herum, zusammengepresst wird.

4. Fertigungsanlage (1) nach einem der vorhergehenden Ansprüche, **dadurch gekennzeichnet, dass** das erste Formatwechselmodul (13) als Bearbeitungsstation (6) für das erste Produktformat und das zweite Formatwechselmodul (13') als Bearbeitungsstation (6') für das zweite Produktformat bezogen auf die Produktionsrichtung (P) zwischen der Schneidevorrichtung (4, 4') und der Lagenverbindevorrichtung (5, 5') jeweils eine Handhabungsvorrichtung (6, 6') aufweist, die konfiguriert ist, die seitlichen Befestigungselemente (15a, 15b) oder Zwischenprodukte (15) in Produktionsrichtung (P) zu beschleunigen und insbesondere in Produktionsrichtung (P) voneinander zu beabstanden und/oder einzelne Befestigungselemente (15a, 15b) oder Zwischenprodukte (15) um insbesondere 180 ° zu drehen.

5. Fertigungsanlage (1) nach einem der vorhergehenden Ansprüche, **dadurch gekennzeichnet, dass** das erste Formatwechselmodul (13) als Bearbeitungsstationen (7, 8, ...12) für das erste Produktformat und das zweite Formatwechselmodul (13') als Bearbeitungsstationen (7', 8',...12') für das zweite Produktformat jeweils aufweist:
- eine erste Zuführvorrichtung (7, 7') konfiguriert zum Zuführen von Bandmaterial (22) für die seitlichen Befestigungselemente (15a, 15b) oder Zwischenprodukte (15), insbesondere in Form von zwei Bandmaterialsträngen (22a, 22b), zur Schneidevorrichtung (4, 4') und/oder
- eine zweite Zuführvorrichtung (8, 8') konfiguriert zum Zuführen des die körperseitige Lage bildenden Bandmaterials (16) zur Lagenverbindevorrichtung (5, 5'), wobei das die körperseitige Lage bildende Bandmaterial (16), insbesondere zumindest mit einer Flüssigkeitsaufnahme- und -verteilungslage (23) und/oder mit Einem Auslaufschutz (24) und/oder mit einer Zwischenlage versehen ist und/oder
- eine dritte Zuführvorrichtung (9, 9') konfiguriert zum Zuführen des die körperferne Lage bildenden Bandmaterials (17) zur Lagenverbindevorrichtung (5, 5'), wobei das die körperferne Lage bildende Bandmaterial (17) insbesondere mit flüssigkeitsabsorbierenden Kernen (20) und/oder mit elastischen Längsbändern (25) versehen ist, und/oder
- eine vierte Zuführvorrichtung (10, 10') konfiguriert zum Zuführen von elastischen Querbändern (26) sowie insbesondere eine Schneidevorrichtung (11, 11'), konfiguriert zum Zuschneiden der elastischen Querbänder (26) und/oder
- eine Übergabevorrichtung (12, 12') konfiguriert zum Übergeben des fixierten Schichtaufbaus (18) an eine weitere Bearbeitungsstation (27, 28, ...33), die insbesondere nicht Bestandteil des jeweiligen Formatwechselmoduls (13, 13') ist.

6. Fertigungsanlage (1) nach einem der vorhergehenden Ansprüche, **dadurch gekennzeichnet, dass** die jeweilige weitere Bearbeitungsstation (27, 28, ...30), die nicht Bestandteil des jeweiligen Formatwechselmoduls (13, 13') ist, dem jeweiligen Formatwechselmodul (13, 13') im in die Fertigungsstraße (14) integrierten Zustand bezogen auf die Produktionsrichtung (P) nachgelagert ist.

7. Fertigungsanlage (1) nach einem der vorhergehenden Ansprüche, **dadurch gekennzeichnet, dass** die Fertigungsanlage (1) als weitere Bearbeitungsstation (31), die nicht Bestandteil des jeweiligen Formatwechselmoduls (13, 13') ist, eine erste Bereitstellungsvorrichtung (31) aufweist, die konfiguriert ist zum Zuführen von Bandmaterial (22) für die seitlichen Befestigungselemente (15a, 15b) oder Zwischenprodukte (15) zum jeweiligen Formatwechselmodul (13, 13') und insbesondere zur ersten Zuführvorrichtung (7, 7') des jeweiligen Formatwechselmoduls (13, 13'), wobei die erste Bereitstellungsvorrichtung (31) insbesondere eine Längszerteilungsvorrichtung (35) zum Zerteilen des Bandmaterials (22) in zwei separate Bandmaterialstränge (22a, 22b) umfasst.

8. Fertigungsanlage (1) nach einem der vorhergehenden Ansprüche, **dadurch gekennzeichnet, dass** die Fertigungsanlage (1) als weitere Bearbeitungsstation (32), die nicht Bestandteil des jeweiligen Formatwechselmoduls (13, 13') ist, eine zweite Bereitstellungsvorrichtung (32) aufweist, die konfiguriert ist zum Zuführen von die körperseitige Lage bildendem Bandmaterial (16) zum jeweiligen Formatwechselmodul (13, 13') und insbesondere zur zweiten Zuführvorrichtung (8, 8') des jeweiligen Formatwechselmoduls (13, 13'), wobei die zweite Bereitstellungsvorrichtung (32) insbesondere eine Schichtungsvorrichtung (36) zum Zusammenführen des die körperseitige Lage bildenden Bandmaterials (16) und einer Flüssigkeitsaufnahme- und -verteilungslage (23) und/oder einem Auslaufschutz (24) und/oder einer Zwischenlage umfasst.

9. Fertigungsanlage (1) nach einem der vorhergehenden Ansprüche, **dadurch gekennzeichnet, dass** die Fertigungsanlage (1) als weitere Bearbeitungsstation eine dritte Bereitstellungsvorrichtung (33) aufweist, die konfiguriert ist zum Zuführen von die körperferne Lage bildendem Bandmaterial (17) zum jeweiligen Formatwechselmodul (13, 13') und insbesondere zur dritten Zuführvorrichtung (9, 9') des jeweiligen Formatwechselmoduls (13, 13 '), wobei die dritte Bereitstellungsvorrichtung (33) insbesondere eine Kernformvorrichtung (37) umfasst, die konfiguriert ist zum Herstellen und Formen flüssigkeitsabsorbierender Kerne (20) und Auflegen der Kerne (20) auf das die körperferne Lage bildende Bandmaterial (17).

10. Fertigungsanlage (1) nach einem der Ansprüche 7 bis 9, **dadurch gekennzeichnet, dass** die jeweilige weitere Bearbeitungsstation (31, 32, 33), die nicht Bestandteil des jeweiligen Formatwechselmoduls (13, 13') ist, dem jeweiligen Formatwechselmodul (13, 13') im in die Fertigungsstraße (14) integrierten Zustand bezogen auf die Produktionsrichtung (P) vorgelagert ist.

11. Fertigungsanlage (1) nach einem der vorhergehenden Ansprüche, **dadurch gekennzeichnet, dass** das jeweilige Formatwechselmodul (13, 13') einen Modulrahmen (37, 37') aufweist, in den die Bearbeitungsstationen (4, 5, ...12; 4', 5', ...12') für das jeweilige Produktformat eingebaut sind, wobei der Modulrahmen (37) des ersten Formatwechselmoduls (13) insbesondere dieselben Abmessungen wie der Modulrahmen (37') des zweiten Formatwechselmoduls (13') aufweist.

12. Fertigungsanlage (1) nach einem der vorhergehenden Ansprüche, **dadurch gekennzeichnet, dass** das jeweilige Formatwechselmodul (13, 13') eine Bewegungseinheit (38, 38') aufweist, die insbesondere Transportrollen oder Luftgleitkissen (39, 39') enthält, und/oder eine Kupplungseinheit (40, 40') konfiguriert zum Zusammenkuppeln mit entsprechenden Gegenstücken (41) in der Fertigungsstraße (14).

## Claims

1. Production plant (1) for hygiene products (2), in particular adult or baby diapers (2),
- comprising a plurality of processing stations (4, 5, ...12) which are configured for producing a first product format and
- comprising a plurality of processing stations (4', 5', ...12') which are configured for producing a second product format,
wherein the processing stations (4, 5, ...12) for the first product format are combined in a first format change module (13) and the processing stations (4', 5',...12' ) for the second product format are combined in a second format change module (13') and
wherein the first format change module (13) and the second format change module (13') can be alternately integrated into a production line (14) of the production plant (1),
wherein the first format change module (13) has as processing stations (4, 5) for the first product format and the second format change module (13') has as processing stations (4', 5') for the second product format in each case:
- a cutting device (4, 4') configured for forming lateral fastening elements (15a, 15b) of the hygiene product (2) or intermediate products (15) from which the lateral fastening elements (15a, 15b) are created, and
- a sheet-connecting device (5, 5') configured for bringing together at least one out of a strip material (16) forming a body-side sheet and a strip material (17) forming the body-remote sheet and the lateral fastening elements (15a, 15b) or intermediate products (15) and connecting them to form a fixed layered structure (18), wherein the lateral fastening elements (15a, 15b) or intermediate products (15) can be connected to the strip material (16) forming the body-side sheet and/or the strip material (17) forming the body-remote sheet, in particular by being integrally bonded, preferably by means of applying hot glue, ultrasonic sealing and/or stamping, frictionally connected and/or interlockingly connected,
wherein the production plant (1) has as a further processing station (27), which does not form part of the respective format change module (13, 13'),
- a first folding device (27), which is configured for longitudinally folding the fixed layered structure (18), and/or
- a cutting-to-length device (28), which is configured for cutting the fixed layered structure (18) to length, and/or
- a second folding device (29), which is configured for transversely folding individual products (34) created from the fixed layered structure (18), and/or
- a stacking device (30), which is configured for stacking, and in particular packing, individual products (34) created from the fixed layered structure (18).

2. Production plant (1) according to Claim 1, **characterized in that** the sheet-connecting device (5, 5') has a collecting drum (19), which is configured to bring together the strip material (16) forming the body-side sheet, which in particular is provided at least with the lateral fastening elements (15a, 15b) or intermediate products (15), with the strip material (17) forming the body-remote sheet, which in particular is provided at least with liquid-absorbing cores (20), wherein the collecting drum (19) is in particular configured to draw the strip material (16) forming the body-side sheet onto it by suction.

3. Production plant (1) according to Claim 2, **characterized in that** the sheet-connecting device (5, 5') has a pressing roller (21), which interacts with the collecting drum (19) in such a way that the layered structure (18) is pressed together, at least in some portions, between the collecting drum (19) and the pressing roller (21), in particular at least around the cores (20).

4. Production plant (1) according to one of the preceding claims, **characterized in that** the first format change module (13) has as a processing station (6) for the first product format and the second format change module (13') has as a processing station (6') for the second product format, between the cutting device (4, 4') and the sheet-connecting device (5, 5') with respect to the direction of production (P), in each case a handling device (6, 6'), which is configured to accelerate the lateral fastening elements (15a, 15b) or intermediate products (15) in the direction of production (P) and in particular to space them apart in the direction of production (P) and/or to turn individual fastening elements (15a, 15b) or intermediate products (15) by in particular 180°.

5. Production plant (1) according to one of the preceding claims, **characterized in that** the first format change module (13) has as processing stations (7, 8, ...12) for the first product format and the second format change module (13') has as processing stations (7', 8',...12') for the second product format in each case:
- a first feeding device (7, 7'), configured for feeding strip material (22) for the lateral fastening elements (15a, 15b) or intermediate products (15), in particular in the form of two strands of strip material (22a, 22b), to the cutting device (4, 4') and/or
- a second feeding device (8, 8'), configured for feeding the strip material (16) forming the body-side sheet to the sheet-connecting device (5, 5'), wherein the strip material (16) forming the body-side sheet is in particular provided at least with a liquid-absorbing and distributing sheet (23) and/or with a leakage preventer (24) and/or with an intermediate sheet and/or
- a third feeding device (9, 9'), configured for feeding the strip material (17) forming the body-remote sheet to the sheet-connecting device (5, 5'), wherein the strip material (17) forming the body-remote sheet is in particular provided with liquid-absorbing cores (20) and/or with elastic longitudinal strips (25), and/or
- a fourth feeding device (10, 10'), configured for feeding elastic transverse strips (26), and also in particular a cutting device (11, 11'), configured for cutting the elastic transverse strips (26) to size, and/or
- a transfer device (12, 12'), configured for transferring the fixed layered structure (18) to a further processing station (27, 28, ...33), which in particular does not form part of the respective format change module (13, 13').

6. Production plant (1) according to one of the preceding claims, **characterized in that** the respective further processing station (27, 28, ...30), which does not form part of the respective format change module (13, 13'), is arranged downstream of the respective format change module (13, 13') with respect to the direction of production (P) in the state in which it is integrated in the production line (14).

7. Production plant (1) according to one of the preceding claims, **characterized in that** the production plant (1) has as a further processing station (31), which does not form part of the respective format change module (13, 13'), a first supplying device (31), which is configured for feeding strip material (22) for the lateral fastening elements (15a, 15b) or intermediate products (15) to the respective format change module (13, 13') and in particular to the first feeding device (7, 7') of the respective format change module (13, 13'), wherein the first supplying device (31) in particular comprises a longitudinal-slicing device (35) for slicing the strip material (22) into two separate strands of strip material (22a, 22b).

8. Production plant (1) according to one of the preceding claims, **characterized in that** the production plant (1) has as a further processing station (32), which does not form part of the respective format change module (13, 13'), a second supplying device (32), which is configured for feeding strip material (16) forming the body-side sheet to the respective format change module (13, 13') and in particular to the second feeding device (8, 8') of the respective format change module (13, 13'), wherein the second supplying device (32) in particular comprises a layering device (36) for bringing together the strip material (16) forming the body-side sheet and a liquid-absorbing and distributing sheet (23) and/or a leakage preventer (24) and/or an intermediate sheet.

9. Production plant (1) according to one of the preceding claims, **characterized in that** the production plant (1) has as a further processing station a third supplying device (33), which is configured for feeding strip material (17) forming the body-remote sheet to the respective format change module (13, 13') and in particular to the third feeding device (9, 9') of the respective format change module (13, 13'), wherein the third supplying device (33) in particular comprises a core-forming device (37), which is configured for producing and forming liquid-absorbing cores (20) and placing the cores (20) onto the strip material (17) forming the body-remote sheet.

10. Production plant (1) according to one of Claims 7 to 9, **characterized in that** the respective further processing station (31, 32, 33), which does not form part of the respective format change module (13, 13'), is arranged upstream of the respective format change module (13, 13') in the direction of production (P) in the state in which it is integrated in the production line (14).

11. Production plant (1) according to one of the preceding claims, **characterized in that** the respective format change module (13, 13') has a module frame (37, 37'), in which the processing stations (4, 5, ...12; 4' , 5', ...12') for the respective product format are installed, wherein the module frame (37) of the first format change module (13) in particular has the same dimensions as the module frame (37') of the second format change module (13').

12. Production plant (1) according to one of the preceding claims, **characterized in that** the respective format change module (13, 13') has a moving unit (38, 38'), which in particular includes transporting rollers or air cushions (39, 39'), and/or a coupling unit (40, 40'), configured for coupling together with corresponding counterparts (41) in the production line (14).

## Revendications

1. Installation de fabrication (1) pour des produits d'hygiène (2), en particulier des couches pour adultes ou pour bébés (2),
- comprenant plusieurs postes de traitement (4, 5,... 12) configurés pour la fabrication d'un premier format de produit, et
- comprenant plusieurs postes de traitement (4', 5',... 12') configurés pour la fabrication d'un deuxième format de produit,
les postes de traitement (4, 5,... 12) pour le premier format de produit étant regroupés dans un premier module (13) de changement de format et les postes de traitement (4', 5, ... 12') pour le deuxième format de produit étant regroupés dans un deuxième module (13') de changement de format, et
le premier module (13) de changement de format et le deuxième module (13') de changement de format étant aptes à être intégrés en alternance dans une ligne de production (14) de l'installation de fabrication (1),
le premier module (13) de changement de format, en tant que postes de traitement (4, 5) pour le premier format de produit, et le deuxième module (13') de changement de format, en tant que postes de traitement (4', 5') pour le deuxième format de produit, comprenant chacun :
- un dispositif de coupe (4, 4') conçu de manière à façonner les éléments de fixation latéraux (15a, 15b) du produit d'hygiène (2) ou des produits intermédiaires (15) à partir desquels les éléments de fixation latéraux (15a, 15b) sont fabriqués, et
- un dispositif (5, 5') de liaison de couches conçu de manière à réunir au moins un matériau en bande (16) formant une couche côté corps, un matériau en bande (17) formant la couche éloignée du corps ainsi que les éléments de fixation latéraux (15a, 15b) ou des produits intermédiaires (15) et pour les assembler en une structure stratifiée fixe (18), les éléments de fixation latéraux (15a, 15b) ou les produits intermédiaires (15) étant aptes à être reliés au matériau en bande (16) formant la couche côté corps et/ou au matériau en bande (17) formant la couche éloignée du corps, en particulier par liaison de matière, de préférence par application de colle chaude, par soudage par ultrasons et/ou par estampage à chaud, par liaison de force et/ou par complémentarité de forme,
l'installation de fabrication (1) comprenant, en tant que poste de traitement supplémentaire (27) qui ne fait pas partie du module (13, 13') de changement de format respectif,
- un premier dispositif de pliage (27) conçu de manière à plier longitudinalement la structure stratifiée fixée (18), et/ou
- un dispositif de coupe (28) conçu de manière à couper la structure stratifiée fixée (18), et/ou
- un deuxième dispositif de pliage (29) conçu de manière à plier transversalement des produits individuels (34) fabriqués à partir de la structure stratifiée fixée (18), et/ou
un dispositif d'empilage (30) conçu de manière à empiler, et en particulier emballer, des produits individuels (34) fabriqués à partir de la structure stratifiée fixe (18).

2. Installation de fabrication (1) selon la revendication 1, **caractérisée en ce que** le dispositif (5, 5') de liaison de couches comprend un tambour collecteur (19) qui est conçu de manière à réunir le matériau en bande (16) formant la couche côté corps, qui est notamment pourvu au moins des éléments de fixation latéraux (15a, 15b) ou des produits intermédiaires (15) avec le matériau en bande (17) formant la couche éloignée du corps, qui est notamment pourvu au moins de noyaux (20) absorbant les liquides, le tambour collecteur (19) étant notamment conçu de manière à aspirer le matériau en bande (16) formant la couche côté corps.

3. Installation de fabrication (1) selon la revendication 2, **caractérisée en ce que** le dispositif (5, 5') de liaison de couches comprend un rouleau de pression (21) qui coopère avec le tambour collecteur (19) de telle manière que la structure en couches (18) soit comprimée au moins partiellement entre le tambour collecteur (19) et le rouleau de pression (21), en particulier au moins autour des noyaux (20).

4. Installation de fabrication (1) selon l'une des revendications précédentes, **caractérisée en ce que** le premier module (13) de changement de format, en tant que poste de traitement (6) pour le premier format de produit, et le deuxième module (13') de changement de format, en tant que poste de traitement (6') pour le deuxième format de produit, par rapport au sens de production (P) entre le dispositif de coupe (4, 4') et le dispositif (5, 5') de liaison de couches, présentent chacun un dispositif de manipulation (6, 6') qui est conçu de manière à accélérer les éléments de fixation latéraux (15a, 15b) ou les produits intermédiaires (15) dans le sens de la production (P), et en particulier à les espacer les uns des autres dans le sens de la fabrication (P) et/ou à faire pivoter certains éléments de fixation (15a, 15b) ou produits intermédiaires (15), en particulier de 180°.

5. Installation de fabrication (1) selon l'une des revendications précédentes, **caractérisée en ce que** le premier module (13) de changement de format, en tant que postes de traitement (7, 8,... 12) pour le premier format de produit, et le deuxième module (13') de changement de format, en tant que postes de traitement (7', 8',... 12') pour le deuxième format de produit, comprennent chacun :
- un premier dispositif d'alimentation (7, 7') conçu de manière à alimenter le dispositif de coupe (4, 4') en bande (22) pour les éléments de fixation latéraux (15a, 15b) ou les produits intermédiaires (15), en particulier sous la forme de deux brins de matériau (22a, 22b), et/ou
- un deuxième dispositif d'alimentation (8, 8') conçu de manière à alimenter la bande (16) formant la couche côté corps vers le dispositif (5, 5') de liaison de couches, le matériau en bande (16) formant la couche côté corps étant notamment pourvu d'au moins une couche (23) d'absorption et de répartition de liquide et/ou d'une protection antifuite (24) et/ou d'une couche intermédiaire, et/ou
- un troisième dispositif d'alimentation (9, 9') conçu de manière à alimenter vers le dispositif (5, 5') de liaison de couches la bande (17) formant la couche éloignée du corps, la bande (17) formant la couche éloignée du corps étant notamment pourvue de noyaux (20) absorbant les liquides et/ou de bandes longitudinales élastiques (25), et/ou
- un quatrième dispositif d'alimentation (10, 10') conçu de manière à alimenter des bandes transversales élastiques (26) ainsi qu'un dispositif de coupe (11, 11') conçu de manière à couper les bandes transversales élastiques (26) et/ou
- un dispositif de transfert (12, 12') conçu de manière à transférer la structure stratifiée fixée (18) vers un autre poste de traitement (27, 28,... 33), qui ne fait notamment pas partie du module de changement de format respectif (13, 13').

6. Installation de fabrication (1) selon l'une des revendications précédentes, **caractérisée en ce que** le poste de traitement supplémentaire (27, 28, ... 30), qui ne fait pas partie du module respectif (13, 13') de changement de format, est placé en aval du module respectif (13, 13') de changement de format dans le sens de la fabrication (P) lorsqu'il est intégré dans la ligne de fabrication (14).

7. Installation de fabrication (1) selon l'une des revendications précédentes, **caractérisée en ce que** l'installation de fabrication (1) comprend, en tant que poste de traitement supplémentaire (31) qui ne fait pas partie du module respectif (13, 13') de changement de format, un premier dispositif de fourniture (31) qui est conçu de manière à alimenter du matériau en bande (22) pour les éléments de fixation latéraux (15a, 15b) ou des produits intermédiaires (15) vers le module respectif (13, 13') de changement de format, et en particulier vers le premier dispositif d'alimentation (7, 7') du module respectif (13, 13') de changement de format, le premier dispositif de fourniture (31) comprenant en particulier un dispositif de division longitudinale (35) pour diviser le matériau en bande (22) en deux brins de matériau en bande séparés (22a, 22b).

8. Installation de fabrication (1) selon l'une des revendications précédentes, **caractérisée en ce que** l'installation de fabrication (1) comprend, en tant que poste de traitement supplémentaire (32) qui ne fait pas partie du module respectif (13, 13') de changement de format, un deuxième dispositif de fourniture (32) qui est conçu de manière à alimenter le matériau en bande (16) formant la couche côté corps vers le module respectif (13, 13') de changement de format, et en particulier vers le deuxième dispositif d'alimentation (8, 8') du module respectif (13, 13') de changement de format, le deuxième dispositif de fourniture (32) comprenant en particulier un dispositif de stratification (36) pour réunir le matériau en bande (16) formant la couche côté corps et une couche de réception et de distribution de liquide (23) et/ou une protection de sortie (24) et/ou une couche intermédiaire.

9. Installation de fabrication (1) selon l'une des revendications précédentes, caractérisée en que l'installation de fabrication (1) comprend, en tant que poste de traitement supplémentaire, un troisième dispositif de fourniture (33) qui est conçu de manière à alimenter le matériau en bande (17) formant la couche éloignée du corps vers le module respectif (13, 13') de changement de format, et en particulier vers le troisième dispositif d'alimentation (9, 9') du module respectif (13, 13') de changement de format, le troisième dispositif de fourniture (33) comprenant en particulier un dispositif (37) de formage de noyaux qui est configuré pour fabriquer et former des noyaux (20) absorbant les liquides et pour placer les noyaux (20) sur le matériau en bande (17) formant la couche éloignée du corps.

10. Installation de fabrication (1) selon l'une des revendications 7 à 9, **caractérisée en ce que** le poste de traitement supplémentaire (31, 32, 33), qui ne fait pas partie du module (13, 13') de changement de format, est placé en amont du module respectif (13, 13') de changement de format intégré dans la ligne de production (14) par rapport au sens de production (P).

11. Installation de fabrication (1) selon l'une des revendications précédentes, **caractérisée en ce que** le module respectif (13, 13') de changement de format comprend un châssis (37, 37') de module dans lequel sont installées les postes de traitement (4, 5,... 12 ; 4', 5',... 12') pour le format de produit respectif, le châssis (37) de module du premier module (13) de changement de format ayant en particulier les mêmes dimensions que le châssis (37') de module du deuxième module (13') de changement de format.

12. Installation de fabrication (1) selon l'une des revendications précédentes, **caractérisée en ce que** le module (13, 13') de changement de format respectif comprend une unité de déplacement (38, 38') qui contient en particulier des rouleaux de transport ou des coussins d'air (39, 39') et/ou une unité de liaison (40, 40') conçue de manière à venir en accouplement avec des contreparties correspondantes (41) dans la ligne de fabrication (14).
